(19) Europäisches Patentamt
European Patent Office
Office européen des brevets

(11) Veröffentlichungsnummer: **0 120 439**
**B1**

(12) # EUROPÄISCHE PATENTSCHRIFT

(45) Veröffentlichungstag der Patentschrift: **14.01.87**

(21) Anmeldenummer: **84103031.5**

(22) Anmeldetag: **20.03.84**

(51) Int. Cl.⁴: **C 07 D 495/14**, C 07 D 333/54, A 61 K 31/435 // (C07D495/14, 209:00, 221:00, 333:00)

(54) **7,8,9,10-Tetrahydrothieno-(3,2-e)-pyrido-(4,3-b)-indole, Verfahren zu ihrer Herstellung und diese enthaltende Arzneimittel.**

(30) Priorität: **29.03.83 DE 3311342**

(43) Veröffentlichungstag der Anmeldung: **03.10.84 Patentblatt 84/40**

(45) Bekanntmachung des Hinweises auf die Patenterteilung: **14.01.87 Patentblatt 87/3**

(84) Benannte Vertragsstaaten: **AT BE CH DE FR GB IT LI LU NL SE**

(56) Entgegenhaltungen:
**DD-A-147 668**

**CHEMICAL ABSTRACTS, vol. 75, no. 7, 16. August 1971, Columbus, Ohio, USA, ABRAMENKO, P.I., ZHIRYAKOV, V.G. "Synthesis of 7,8,8-trimethyl-thieno(3,2-e)indolenine. Seite 355, Spalte 2, Abstract Nr. 48 953p**
**CHEMICAL ABSTRACTS, vol. 80, no. 18, 6. Mai 1974, Columbus, Ohio, USA, KAMEL, M.; ALLAM, M.A.; AL-AREF, S.; AL-AREF, A.T. "New monoazo dyes containing the benzo(b)thiophene nucleus", Seite 73, Spalte 2, Abstract Nr. 97 319y**

(73) Patentinhaber: **Troponwerke GmbH & Co. KG, Berliner Strasse 156, D-5000 Köln 80 (DE)**

(72) Erfinder: **Seidel, Peter- Rudolf, Dr., Alte Heide 5d, D-5000 Köln 90 (DE)**
Erfinder: **Schöllnhammer, Günter, Dr., Hackberg 21, D-5060 Bergisch- Gladbach (DE)**

(74) Vertreter: **Mann, Volker, Dr., c/o Bayer AG Konzernverwaltung RP Patentabteilung, D-5090 Leverkusen 1, Bayerwerk (DE)**

EP 0 120 439 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue substituierte 7, 8, 9, 10-Tetrahydrothieno [3,2-e] pyrido [4,3-b] indole, ein Verfahren zu ihrer Herstellung sowie diese enthaltende Arzneimittel, ihre Verwendung als Arzneimittel, insbesondere als das zentrale Nervensystem beeinflussende Mittel.

Einige 7, 8, 9, 10-Tetrahydrothieno [3,2-e] pyrido [4,3-b]-indole und ihre zentralnervösen Wirkungen, insbesondere ihre antidepressiven Wirkungen, werden bereits in der Europäischen Patentschrift 12 347, allgemein beschrieben. Die Wirkung dieser Verbindungen ist jedoch nicht immer voll befriedigend.

Es wurde nun gefunden, daß die neuen substituierten 7, 8, 9, 10-Tetrahydrothieno [3,2-e] pyrido [4,3-b]indole der allgemeinen Formel I,

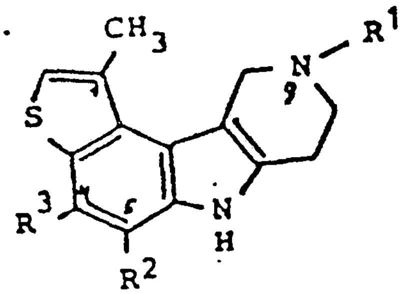

(I)

in welcher

$R^1$ für Wasserstoff oder geradkettigen oder verzweigter Alkyl mit 1 bei C-Atomen steht, und

$R^2$ und $R^3$ für ein Wasserstoff und ein Halogen oder für zwei gleiche oder verschiedene Halogene stehen,

sowie deren Säureadditionssalze gute zentralnervöse, insbesondere antidepressive Eigenschaften aufweisen.

Überraschenderweise zeigen die neuen erfindungsgemäß zu verwendenden substituierten 7, 8, 9, 10-Tetrahydrothieno-[3,2-e] pyrido [4,3-b] indol-Derivate der Formel (I) ein besseres zentralnervöses Wirkungsspektrum, insbesondere auch eine bessere therapeutisch nutzbare in-vivo-Wirksamkeit als die aus dem Stand der Technik bekannten und in der Europäischen Patentschrift 12 347 beschriebenen Verbindungen. Die erfindungsgemäßen Verbindungen der Formel (I) sowie ihre pharmazeutische Verwendung stellt somit eine Bereicherung der Pharmazie dar.

Die erfindungsgemäßen substituierten 7, 8, 9, 10-Tetrahy-drothieno [3,2-e] pyrido [4,3-b] indol-Derivate sind durch die Formel (I) allgemein definiert. Bevorzugt sind diejenigen Verbindungen der Formel (I), in denen

$R^1$ für Wasserstoff, geradkettiges oder verzweigtes Alkyl ($C_1$ - $C_4$) steht, und

$R^3$ und $R^4$ in der Bedeutung Halogen für Fluor oder Chlor stehen.

Die vorliegende Erfindung betrifft weiterhin ein Verfahren zur Herstellung von Verbindungen der allgemeinen Formel (I), dadurch gekennzeichnet, daß man Hydrazinverbindungen der allgemeinen Formel (II)

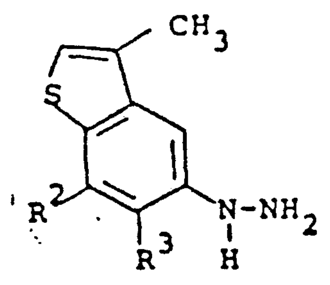

(II).

in welcher

$R^2$ und $R^3$ die oben angegebene Bedeutung haben,

mit Piperidonen der allgemeinen Formel (III)

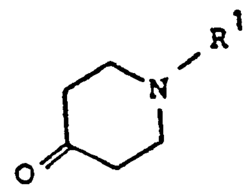

(III)

in welcher
$R^1$ die oben angegebene Bedeutung hat
bzw. mit Salzen dieser Piperidone mit anorganischen oder organischen Lösungsmitteln bei Temperaturen zwischen 20 und 250°C, gegebenenfalls in Gegenwart von Kondensationsmitteln, umgesetzt und die so erhaltenen Verbindungen der Formel (I) gegebenenfalls anschließend in bekannter Weise in die Säureadditionssalze überführt.

Die neuen Verbindungen der allgemeinen Formel (I) zeigen überraschenderweise ausgeprägte und vorteilhafte Wirkungen auf das zentrale Nervensystem. Besonders erwähnt sei ihre Verwendung als Antidepressiva.

Vorzug dieser neuen Verbindungen ist, daß sie an den mit $R^2$ und $R^3$ bezeichneten Substitutionsstellen, wobei $R^2$ und $R^3$ die oben angegebene Bedeutung haben, nicht mehr durch Hydroxylierung metabolisiert werden können.

Von besonderer Bedeutung und erfindungsgemäß besonders bevorzugt, sind Verbindungen der allgemeinen Formel (I) in welcher
$R^1$ für Wasserstoff, Methyl, Ethyl, Propyl, i-Propyl, n-Butyl, i-Butyl oder t-Butyl steht, und
$R^2$ und $R^3$ in der Bedeutung Halogen für Fluor stehen.

Ganz besonders bevorzugt sind Verbindungen der allgemeinen Formel (I), in welcher
$R^1$ für Methyl oder Ethyl steht,
$R^2$ für Fluor steht, und
$R^3$ für Wasserstoff steht.

Ein wesentlicher Bestandteil dieser Erfindung sind ebenfalls die zur Herstellung der Verbindungen der allgemeinen Formel (I) benötigten Ausgangsverbindungen der Formel (II) und ihre Darstellung.

Bei der Herstellung der erfindungsgemäßen Verbindungen der allgemeinen Formel (I) aus Hydrazinverbindungen der allgemeinen Formel (II) und Piperidonen der allgemeinen Formel (III) nach folgendem Schema:

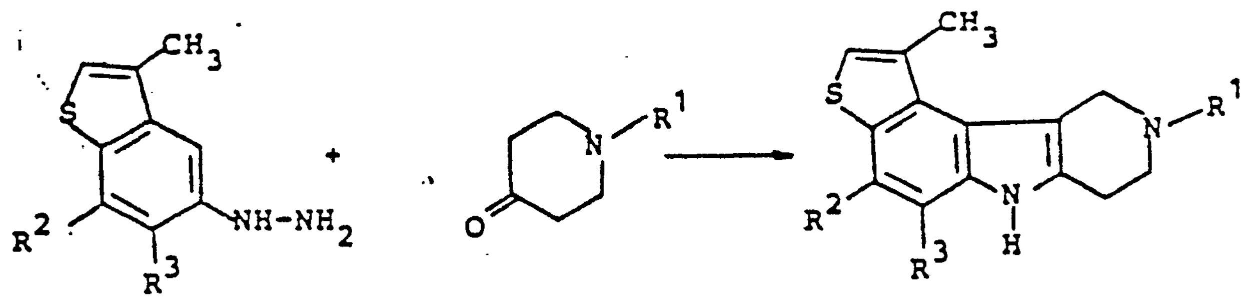

II III I

kann je nach Reaktivität der Reaktionspartner nach zwei verschiedenen Varianten verfahren werden:

Variante (a): Bei den säureunempfindlichen Reaktionspartnern (II und III) setzt man deren Salze, vorzugsweise die Hydrochloride, in einem geeigneten Verdünnungsmittel ein.

Variante (b): Bei Reaktionspartnern (II und III), die sich bevorzugt in Form ihrer Basen zu Verbindungen der Formel (I) umsetzen oder die sich durch Säureempfindlichkeit auszeichnen, arbeitet man vorzugsweise bei höheren Temperaturen in Gegenwart von hochsiedenden Lösungsmitteln oder ohne Lösungsmittel.

Bei beiden Reaktionsvarianten ist es vorteilhaft, die Reaktionen unter Inertgasatmosphäre, wie z.B. Stickstoff oder Argon, durchzuführen.

Als Lösungsmittel für die Verfahrensvariante (a) eignen sich alle Lösungsmittel, die für die Fischer-Indol-Synthese üblich sind (vgl. E. Enders, Houben-Weyl, Band 10/2, S. 546-586, 1967; A. Weissberger, The Chemistry of Heterocyclic Compounds, Indole, Part I, S. 232-370, 1972).

Beispielhaft seien genannte: Wasser, Methanol, Ethanol, Propyl-, Isopropylalkohol, Benzol, Toluol, Xylol,

Dioxan, Eisessig, Propionsäure, Polyphosphorsäureethylester oder hochsiedende Kohlenwasserstoffe.

Als Kondensationsmittel werden ebenfalls die für den Indolringschluß üblichen Katalysatoren verwendet. Beispielhaft seien genannte: Zinkchlorid, Bortrifuorid, Bortrifluoridetherat, Chlorwasserstoff, Salzsäure, Schwefelsäure, Phosphorsäure, Polyphosphorsäure, Polyphosphorsäureethylester, Ameisensäure, Trifluoressigsäure, saure Ionenaustauscher wie z.B. Amberlite oder ein Gemisch aus Eisessig-Chlorwasserstoff.

Die Reaktionstemperaturen können in einem größeren Bereich variiert werden. Im allgemeinen arbeitet man zwischen 40°C und 150°C, vorzugsweise zwischen 60°C und 120°C. Die Reaktionszeiten bewegen sich zwischen 0,5 und 20 Stunden je nach Reaktionstemperatur.

Die Umsetzung wird normalerweise bei Atmosphärendruck durchgeführt.

Bei der Durchführung des erfindungsgemäßen Verfahrens (a) setzt man vorteilhaft auf ein Mol der Hydrazinverbindung (II) das Piperidon (III) in einem Überschuß von 0,1 bis 0,5 Mol ein.

Zweckmäßigerweise kann die Umsetzung auch unter Inertgas, wie z.B. Stickstoff oder Argon, durchgeführt werden. Das Keton der allgemeinen Formel (III) kann in einzelnen Fällen vorteilhaft auch als entsprechendes Ketal, wie z.B. Ethylenketal oder Propylenketal, eingesetzt werden.

Die Aufarbeitung erfolgt zweckmäßig durch Eindampfen der Reaktionslösung, Aufnehmen des Konzentrats in einem geeigneten inerten organischen Lösungsmittel, Alkalisieren mit einer Base, z.B. NaOH oder $NH_3$, und Reinigen, gegebenenfalls mit Hilfe der Chromatographie an Kieselgel bzw. Aluminiumoxid oder anderen geeigneten Adsorbentien.

Die Verfahrensvariante (b) wird vorzugsweise durchgeführt bei Temperaturen zwischen 150 und 210°C. Die Verwendung von Inertgasatmosphäre ist vorteilhaft. Als Lösungsmittel für diese Reaktionsvariante seien beispielhaft genannt: Tetralin, Dichlorbenzol, Acetamid, Ethylenglykol, Diethylenglykol, Diethylenglykolmonomethylether, Triethylenglykol, Glycerin, N-Methyl-pyrrolidon, Ethylenglykoldimethylether, Diethylenglykoldibutylether, wobei Ethylenglykol besonders bevorzugt genannt sei.

Verbindungen der allgmeinen Formel (I), in denen der Substituent $R^1$ am Stickstoff für Wasserstoff steht, lassen sich nach bekannten Methoden nachträglich in entsprechend substituierte Verbindungen überführen. Eine solche nachträgliche Substitution erfolgt vorzugsweise, indem man in an sich bekannter Weise mit den entsprechend substituierter Halogeniden, wie z.B. Alkylhalogeniden, vorzugsweise Methyljodid, Ethylbromid, Ethyljodid, Propylbromid, Isopropylchlorid, n-Butylbromid, insbesondere Methyljodid, Ethylbromid oder Ethyljodid umsetzt, oder mit den entsprechend substituierten Säurehalogeniden, wie z.B. Acetylbromid oder Acetylchlorid umsetzt und anschließend in an sich bekannter Weise mit komplexen Metallhydriden, vorzugsweise mit Lithiumaluminiumhydrid, reduziert.

Die Acylierung und Alkylierung der NH-Gruppe im Tetrahydropyridinteil von I mit $R^1$ für H kann gegebenenfalls bei ausreichender Basizität dieser Gruppe auch direkt in Gegenwart geeigneter Protonenakzeptoren wie Trimethylamin, Triethylamin, N-Methylmorpholin, N-Methylpiperidin, N,N-Dimethylanilin, N,N-Diethylanilin, heterocyclische Basen, wie Pyridin, Picoline, Kollidine, Chinolin oder Isochinolin durchgeführt werden.

Die Umsetzung kann ohne Lösemittel oder aber in Gegenwart geeigneter Lösungsvermittler durchgeführt werden. Als Lösungsvermittler kommen alle organischen Lösemittel in Frage, die gegenüber den jeweiligen Reaktionspartnern inert sind. Hierzu gehören vorzugsweise aromatische Kohlenwasserstoffe wie Benzol, Toluol, Xylol oder Tetralin, Ether wie Diethylether, Diisopropylether, Tetrahydrofuran, Dioxan, Ethylenglykoldiethylether, Nitrile wie Acetonitril, Propionitril, Carbonsäureamide wie Dimethylformamid, Dimethylacetamid, Hexamethylphosphorsäuretriamid, N-Methylpyrrolidon, Dimethylsulfoxid, heterocyclische Basen wie Pyridin, Chinolin oder Picoline, ferner handelsübliche technische Gemische dieser Lösemittel.

Die Umsetzung kann bei Normaldruck, aber auch bei erhöhtem Druck durchgeführt werden, insbesondere bei niedrig siedenden Alkylhalogniden als Reaktionspartner kann erhöhter Druck für die Umsetzung erforderlich sein.

Die Reaktionstemperaturen können in einem gewissen Bereich variiert werden. Im allgemeinen arbeitet man bei Temperaturen zwischen 0 und 200°C, vorzugsweise zwischen 20 und 150°C, insbesondere zwischen 40 und 80°C, in einzelnen Fällen genügt Raumtemperatur.

Die Aufarbeitung erfolgt dann wieder analog wie unter Verfahrensvariante a) beschrieben.

Die als Vorprodukte einzusetzenden 3-Methyl-5-hydrazino-benzothiophene der allgemeinen Formel (II),

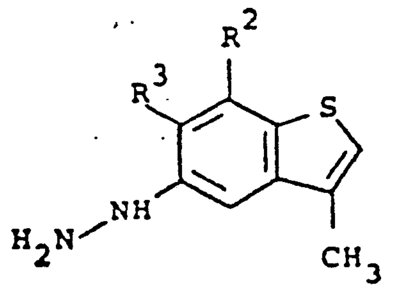

(II)

in welcher
$R^2$ und $R^3$ vorzugsweise in der Bedeutung Halogen für Fluor und/oder Chlor stehen,
sind neu und ebenfalls Gegenstand der vorliegenden Erfindung. Sie lassen sich in allgemein üblicher und bekannter Weise herstellen, indem man die neuen und ebenfalls zum Bestandteil der Erfindung zählenden 3-Methyl-5-nitrobenzothiophene der allgemeinen Formel (IV),

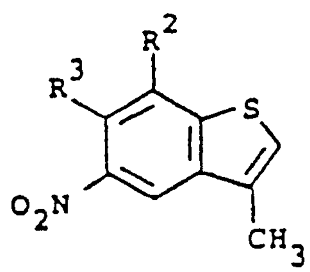

(IV)

in welcher
$R^2$ und $R^3$ die bei der allgemeinen Formel (I) angegebene Bedeutung haben,
mit geeigneten Reduktionsmitteln zu den neuen, ebenfalls zum Bestandteil der Erfindung zählenden 5-Amino-3-methyl-benzothiophenen der allgemeinen Formel (V),

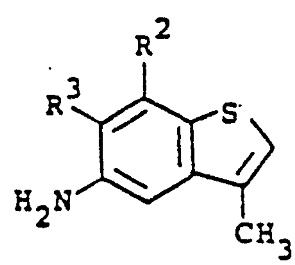

(V)

in welcher
$R^2$ und $R^3$ die in (I) angegebene Bedeutung haben,
reduziert und diese nach bekanntem Verfahren in die Hydrazinverbindungen (II) umwandelt (vgl. E. Enders in: Methoden der Organischen Chemie (Houben-Weyl), Vol. 10, Teil 2: Methoden zur Herstellung und Umwandlung von Arylhydrazinen und Arylhydrazonen, S. 177-406 (1967)):

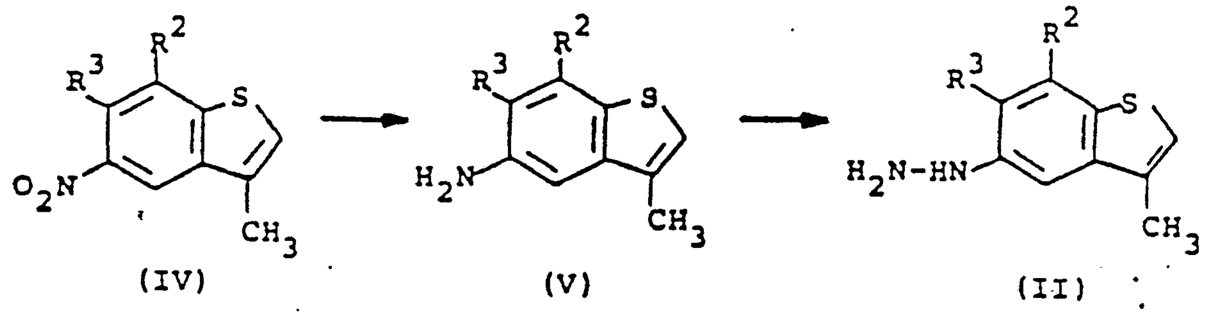

Zur Darstellung von (V) löst man die Nitroverbindung der Formel (IV) in einem geeigneten Lösungsmittel, wie beispielsweise Methanol, Ethanol oder Essigsäureethylester, und hydriert in Gegenwart eines geeigneten Katalysators, wie beispielsweise Palladium, Platin oder Raney-Nickel, in Wasserstoffatmosphäre bei Temperaturen zwischen 20° C und 80° C, vorzugsweise zwischen 20° C und 40° C bei Normaldruck oder erhöhtem Druck; oder man löst die Nitroverbindung (IV) in geeigneten Lösungsmitteln, beispielsweise in Alkoholen, vorzugsweise in Methanol oder Ethanol, fügt überschüssiges Hydrazinhydrat in einem Molverhältnis 1: 5, vorzugsweise in einem Molverhältnis 1: 3, und einen Hydrierkatalysator, beispielsweise Palladium oder Palladiumkohle, hinzu und erhitzt die Mischung 0,5 bis 5 Stunden auf 30° C bis 100° C, vorzugsweise 1 bis 2 Stunden auf 65° C bis 80° C (vgl. N.B. Chapman et al., J. Chem. Soc. (C), 1968, 518; A. Ricci und N. Cagnoli, Ann. Chim. (Rome), 45, 172 (1955); C.A. 50, 5564c (1956)).

Die Aufarbeitung der Reaktionsansätze zur Isolierung der Ausgangsstoffe (II) und Zwischenprodukte (IV) für das erfindungsgemäße Verfahren erfolgt in bekannter Weise.

Die halogensubstituierten 3-Methyl-5-nitrobenzothiophen-Derivate der Formel (IV), in welcher $R^2$ und $R^3$ die oben angegebene Bedeutung haben, sind neu. Ihre Herstellung erfolgt nach an sich bekannten Methoden und wird mit der Synthese des 7-Fluor-3-methyl-5-nitrobenzothiophen (IV-1), entsprechend der allgemeinen Formel IV, in der $R^2$ für F und $R^3$ für H steht, beispielhaft in dem folgenden Reaktionsschema:

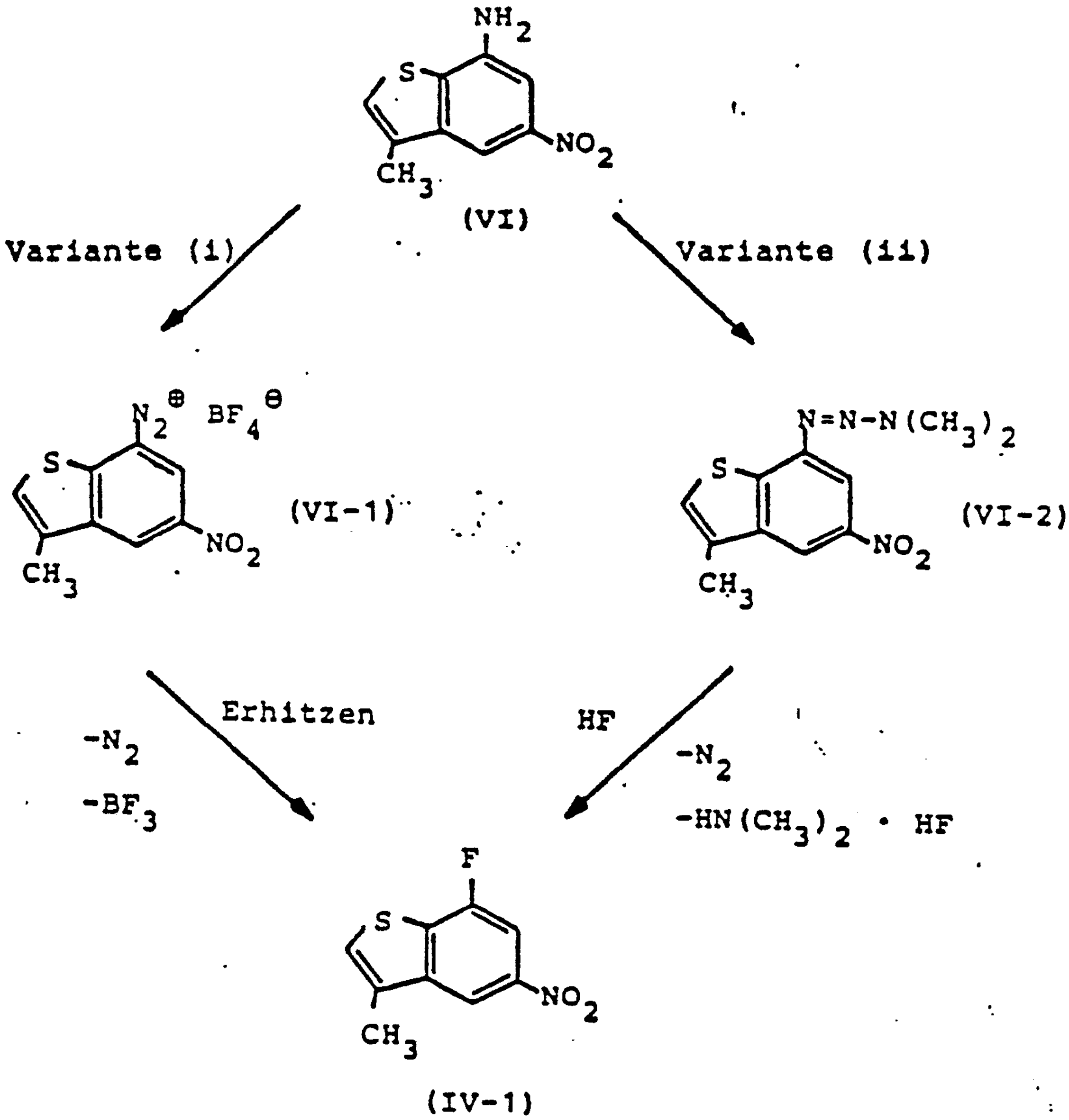


(VI)

(VI-1)

(VI-2)

(IV-1)

Variante (i) besteht aus zwei Reaktionsschritten:

1. Diazotierung der Verbindung (VI) und Herstellung des Diazoniumfluoborats, beispielsweise Verbindung (VI-1). Die als Balz-Schiemann-Reaktion bekannte Methode zur Darstellung kernfluorierter aromatischer Verbindungen gehört als solche zum Stand der Technik (vgl. (a) E. Forche in: Methoden der Organischen Chemie (Houben-Weyl), Vol. 5/3, S. 213-247; Georg Thieme Verlag Stuttgart, 1962;(b) H. Suschitzky in: Stacy/Tatlow/Sharpe, Advances in Fluorine Chemistry, Vol. 4, S. 1 ff; Butterworths London, 1965; (c) G. Schiemann und B. Cornils, Chemie und Technologie cyclischer Fluorverbindungen, S. 9-17, Ferdinand Enke Verlag Stuttgart, 1969).

Variante (ii) beruht auf dem Austausch der Triazen-Gruppe, beispielsweise in (VI-2), durch ein Fluoratom, indem man auf das Triazen-Derivat, das durch Diazotierung von (VI) und anschließende Umsetzung der Diazoniumverbindung mit sekundären Aminen, vorzugsweise Dimethylamin, erhalten wird (vgl. E. Müller in: Methoden der Organischen Chemie (Houben-Weyl), Vol. 10/2, S. 827-835; Georg Thieme Verlag Stuttgart, 1967), Flußsäure, wasserfreien Fluorwasserstoff oder Pyridin-hydrofluorid einwirken läßt (vgl. (a) M.N. Rosenfeld u. D.A. Widdowson, J. Chem. Soc., Chem. Comm. 1979, 914; (b) G. Schiemann u. B. Cornils, Chemie und Technologie cyclischer Fluorverbindungen, S. 8; Ferdinand Enke Verlag Stuttgart, 1969).

Die Ausgangsverbindung (VI) und deren Vorstufen sind neu. Sie können nach an sich bekannten Methoden beispielhaft in der Reaktionsfolge Stufe A → Stufe B → Stufe C → Stufe D → Stufe E hergestellt werden:

Stufe A:

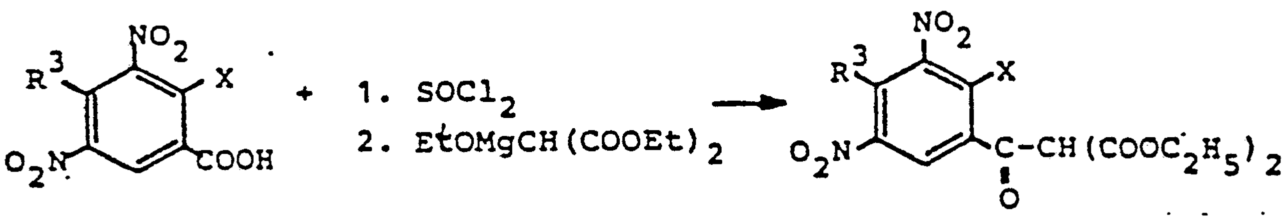

vgl. (a) N.B. Chapman et al., J. Chem. Soc. (C) 1968, 518; (b) A.P.G. Kieboom, Synthesis 1975, 327, (c) S.R. Alpha, J. Org. Chem. 38, 3136 (1973).

Stufe B:

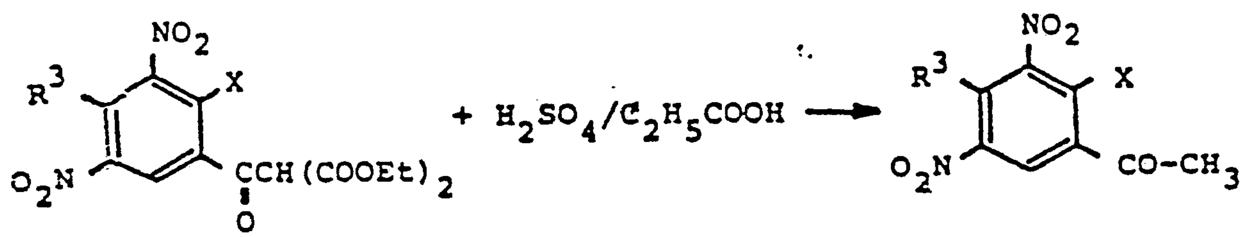

vgl. (a) N.B. Chapman et al., J. Chem. Soc. (C) 1968, 518, (b) H. Henecka in: Methoden der Organischen Chemie (Houben-Weyl), Vol. 7/2b, S. 1338 ff; Georg Thieme Verlag Stuttgart, 1976.

Stufe C:

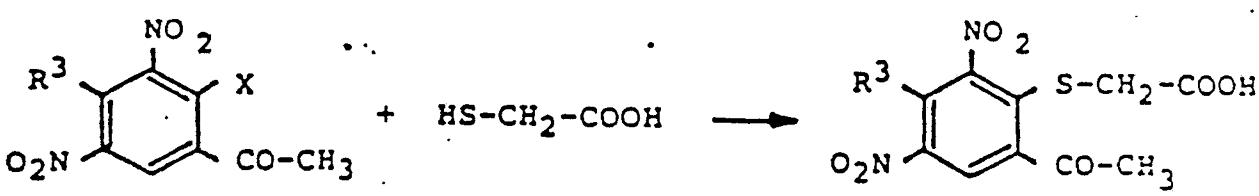

vgl. (a) A Ricci u N Cagnoli Ann. Chim. (Kome) 45, 172 (1955); C.A. 50 5564c (1956); (b) C. Angelini, Ann. Chim. (Rome) 47, 705 (1957); C.A. 52, 1136i (1958); (c) S. Middleton, Austr. J. Chem. 12, 218 (1959).

Stufe D:

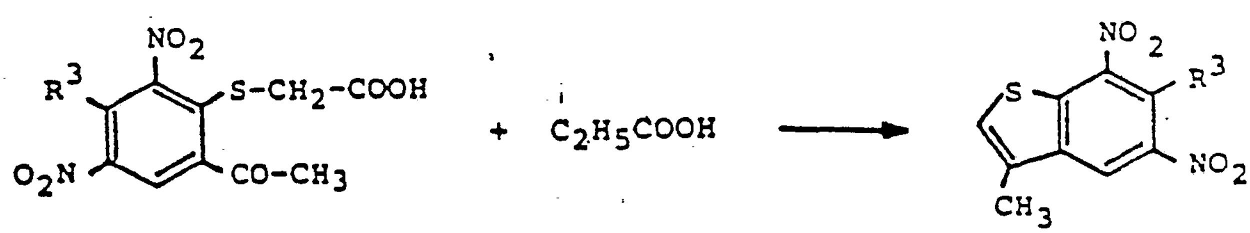

vgl. DE-OS 3 031 738

Stufe E:

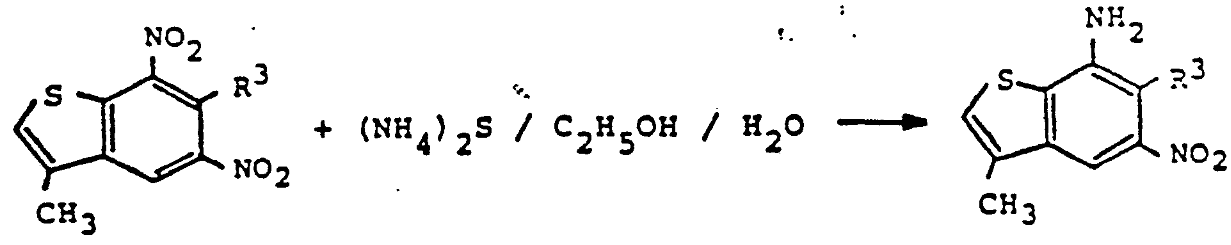


(VI)

vgl. (a) Organ. Synthesis Collect., Vol. III, S. 82-84 (1955); (b) C. Angelini, Ann. Chim. (Rome) 48, 637 (1958); C. A. 53, 5228d (1959).

In den Formeln für die Stufen A bis E hat $R^3$ die oben vorzugsweise angegebene Bedeutung. In den Formeln für die Stufen A bis C steht X für Halogen, insbesondere für Brom, Chlor oder Fluor, vorzugsweise für Chlor.

Als neue Wirkstoffe seien im einzelnen genannt:
1,9-Dimethyl-4-fluor-7,8,9,10-tetrahydrothieno [3,2-e]-pyrido [4,3-b]indol;
1-Methyl-9-ethyl-4-fluor-7,8,9,10-tetrahydrothieno-[3,2-e]-pyrido [4,3-b]indol;
1,9-Dimethyl-5-fluor-7,8,9,10-tetrahydrothieno [3,2-e]-pyrido [4,3-b]indol;
1-Methyl-9-ethyl-5-fluor-7,8,9,10-tetrahydrothieno-[3,2-e]pyrido [4,3-b]indol;
1,9-Dimethyl-4,5-difluor-7,8,9,10-tetrahydrothieno-[3,2-e]pyrido [4,3-b]indol;

Zur vorliegenden Erfindung gehören pharmazeutische Zubereitungen, die neben nicht-toxischen, inerten, pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsjeder Art zu verstehen.

Als bevorzugte pharmazeutische Zubereitungen seien Tabletten, Dragees, Kapseln, Pillen; Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin, und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit, und (i) Gleitmittel, z.B.Ta1kum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett, und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z.B. Wasser, Ethylalkohol, Isopropylalkohol, Ethylcarbonat, Ethylacetat, Benzylalkohol, Benzylbenzoat, Propylenglykol, 1,3-Buty-lenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös appliziert werden.

Im allgemeinen hat es sich als vorteilhaft erwiesen, den oder die Wirkstoffe bei parenteraler (i.v. oder i.m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 30, insbesondere 0,03 bis 3 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Zur vorliegenden Erfindung gehören auch solche Arzneimittel, die neben Verbindungen der Formel (I) noch weitere Wirkstoffe enthalten. Vorzugsweise seien genannt: β-Rezeptorenblocker, Parasympathikolytika, Anxiolytika, Neuroleptika, Hypnotika und Tranquilizer.

Für Beispiel 1 sei die pharmakulogische Wirksamkeit in vier für die Erfassung psychotroper Eigenschaften relevanten Prüfmodellen angegeben.

1. A-phetamin-Potenzierung

Antidepressiv wirksame Substanzen potenzieren das Amphetamin induzierte stereotype Verhalten bei der Ratte. Der angegebene $DE_{50}$-Wert ist die Dosis, bei der das Amphetamininduzierte Verhalten nach Gabe vom 2 mg/kg DL-Amphetaminsulfat i.v. um 50 % verstärkt wird.

Lit: J. L. Howard et al., in: Antidepressants: Neurochemical, Behavioral and Clinical Perspectives, herausgegeben von S. J. Enna et al., Raven Press, New York, S. 107 - 120, 1981

$DE_{50}$ 0,4 mg/kg p.o.

2. Tetrabenazin-Antagonismus

Antidepressiva antagonisieren die durch Tetrabenazin induzierte Ptosis bei Mäusen. Der $DE_{50}$-Wert gibt diejenige Dosis an, bei der die durch Tetrabenazin (20 mg/kg i.p.) induzierte Ptosis zu 50 % reduziert ist.

Lit: J. L. Howard et al., in: Antidepressants Neurochemical, Behavioral and Clinical Perspectives, herausgegeben von S. J. Enna et al., Raven Press, New York, S. 107 - 120, 1981

$DE_{50}$ 5,5 mg/kg.p.o.

3. Anti-aggressive Wirkung

Anxiolytika und Neuroleptika hemmen das durch elektrische Fußschocke evozierte aggressive Verhalten zwischen Mäusen. Der $DE_{50}$-Wert ist die Dosis, bei der das aggressive Verhalten um 50 % reduziert wird.

Lit: Tedeschi et al., J. Pharmacol. Exp. Ther. 129: 28 - 34, 1954.

$DE_{50}$ 7,7 mg/ g i.p.

4. Vermeidungsverhalten

Ratten vermeiden das Betreten eines dunklen Kastens, in dem sie vorher einen elektrischen Fußschock bekommen haben. Dieses Vermeidungsverhalten wird nach Gabe von Anxiolytika aufgehoben. Die niedrigst wirksame Dosis, bei der das Vermeidungsverhalten signifikant reduziert wird, ist angegeben.

Lit: Ader et al., Psychon. Sci: 26: 125 - 128, 1972. Niedrigst wirksame Dosis 2,5 mg/kg i.p.

Die vorliegende Erfindung soll durch die folgenden Beispiele noch näher erläutert werden.

**Beispiel 1**

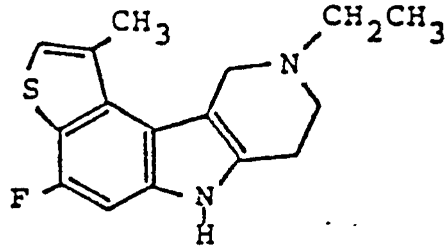

1-Methyl-9-ethyl-4-fluor-7,8,9,10-tetrahydrothieno-[3,2-e]-pyrido[4,3-b]indol

0,1 Mol 3-Methyl-7-fluor-5-hydrazinobenzothiophenhydro-chlorid und 0,11 Mol 1-Ethylpiperidon löst man in der Kälte in 300 ml Isopropanol. Die Lösung wird zum Sieden gebracht und in der Hitze innerhalb von 10 Minuten mit 100 ml HCl-gesättigtem Isopropanol versetzt. Nach 1-stündigem Kochen wird auf 0°C abgekühlt und das entstehende Kristallisat abgesaugt. Zur Reinigung versetzt man das Rohprodukt mit 300 ml 10-%iger Natronlauge, nimmt die Base in Methylenchlorid auf und trocknet die organische Phase nach dem Waschen mit Wasser über Natriumsulfat. Nach dem Filtrieren und Abdampfen des Lösungsmittels wird die Base aus Isopropylether kristallin erhalten.

Ausbeute: 75 % der Theorie; Fp. 182 - 183°C nach Umkristallisieren aus Essigsäureethylester.

Lactat: 0,05 Mol Base löst man in 700 ml Aceton und versetzt mit 15 g L(+)-Milchsäure. Farblose Kristalle. Ausbeute: 95 % der Theorie; Fp. 203-205°C.

Analog wird hergestellt:

1,9-Dimethyl-4-fluor-7,8,9,10-tetrahydrothieno [3,2-e]-pyrido [4,3-b]indol,

Ausbeute: 45 % der Theorie. Fp. 234 - 235 °C nach Umkristallisieren aus Essigsäureethylester.

**Herstellung der Ausgangsprodukte**

3-Methyl-7-fluor-5-hydrazinobenzothiophenhydrochlorid

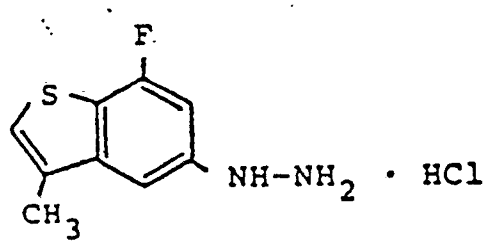

0,1 Mol 3-Methyl-7-fluor-5-aminobenzothiophenhydrochlo-rid werden in 100 ml Wasser und 100 ml konzentrierter Salzsäure suspendiert und zwischen -5°C und 0°C tropfenweise mit einer Lösung aus 0,11 Mol Natriumnitrit in 50 ml Wasser versetzt. Die Diazoniumsalzlösung wird in ein auf 0°C abgekühltes Gemisch aus 0,21 Mol $SnCl_2$. $2H_2O$ und 200 ml konzentrierter Salzsäure eingetropft.

Nach Erwärmen auf Raumtemperatur wird mit 60 ml Isopropanol versetzt, die Kristallmasse abgesaugt und aus Isopropanol umkristallisiert.

Ausbeute: 88 % der Theorie; Fp. 205 - 210°C (Zers.).

3-Methyl-7-fluor-5-aminobenzothiophenhydrochlorid

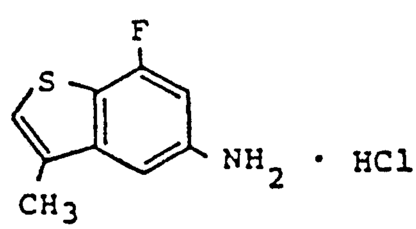

**(Verfahren a)**

0,01 Mol 3-Methyl-7-fluor-5-nitrobenzothiophen werden in 300 ml Methanol in Gegenwart von 1 g Palladiumkohle bei 25°C hydriert. Nach dem Abfiltrieren vom Katalysator engt man das Filtrat auf ca. 100 ml ein, fügt eine Ether-HCl-Lösung hinzu und saugt das Hydrochlorid ab.

Ausbeute: 92 % der Theorie; Fp. 273 - 275°C (Zers.).

**(Verfahren b)**

0,09 Mol 3-Methyl-7-fluor-5-nitrobenzothiophen werden in 220 ml Methanol zusammen mit 2 g Palladiumkohle zum Sieden erhitzt. Anschließend werden 0,35 Mol Hydrazinhydrat innerhalb 30 Minuten zugetropft. Nach 2-stündi-gem Kochen wird filtriert, das Filtrat eingeengt, der Rückstand in Ether gelöst und mit Wasser gewaschen. Aus der Etherlösung wird das Hydrochlorid gefällt.

Ausbeute: 97 % der Theorie; Fp. 273 - 275° C (Zers.).

3-Methyl-7-fluor-5-nitrobenzothiophen

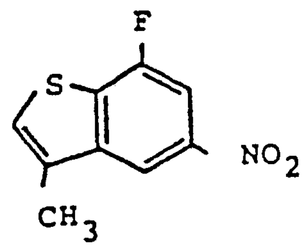

0,5 Mol 1,1-Dimethyl-3-(3-methyl-5-nitrobenzothiophen-7-yl)triazen werden in 10 Mol Flußsäure portionsweise eingetragen. Anschließend verschließt man die Apparatur, preßt 5 bar Stickstoff auf und erwärmt auf 100° C. Der entstehende Stickstoff wird bei 9 bar kontinuierlich entspannt. Nach Reaktionsende (ca. 1 Stunde) wird auf Raumtemperatur abgekühlt, der Restdruck entspannt und überschüssige HF im Vakuum abdestilliert. Den Rückstand rührt man in Methylenchlorid ein, wäscht mit Wasser und abschließend mit $NaHCO_3$-Lösung neutral, trocknet und engt ein. Reinigung erfolgt durch Umkristallisieren aus Leichtbenzin.

Ausbeute: 60 % der Theorie; Fp. 109 - 110° C.

1,1-Dimethyl-3-(3-methyl-5-nitrobenzothiophen-7-yl)triazen

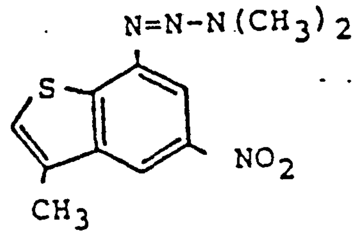

0,05 Mol 3-Methyl-5-nitro-7-aminobenzothiophen werden unter Eiskühlung in 100 ml konzentrierter Schwefelsäure gelöst und bei 10°C durch Zutropfen von 0,05 Mol Nitrosylschwefelsäure diazotiert. Nach 15 Minuten wird der Ansatz auf 700 g Eis gegossen, mit 0,06 Mol Dimethylamin (40 %ige wäßrige Dimethylaminlösung) versetzt und mit 20 %iger Natronlauge alkalisiert. Anschließend wird das Triazen mit Methylenchlorid ausgeschüttelt, die organische Lösung mit Wasser gewaschen und über $Na_2SO_4$ getrocknet. Der nach dem Filtrieren und Abdampfen des Lösungsmittels erhaltene kristalline Rückstand wird in wenig Methylenchlorid aufgenommen und an $Al_2O_3$ chromatographiert (Elutionsmittel: Methylenchlorid / Cyclohexan (1: 1)). Das nach Eindampfen der Eluate erhaltene Produkt wird aus Isopropanol umkristallisiert.

Ausbeute: 63 % der Theorie; Fp. 147 - 148° C.

3-Methyl-5-nitro-7-aminobenzothiophen

(Stufe E)

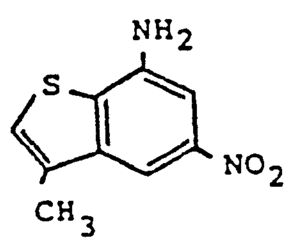

Zu einer Suspension von 0,4 Mol 3-Methyl-5,7-dinitro-benzothiophen in 1.400 ml Ethanol tropft man bei 55-60° C innerhalb 45 Minuten 453 ml einer 20 %igen Ammoniumsulfid-Lösung (1,33 Mol $(NH_4)_2S$) und erhitzt anschließend 15 Minuten lang zum Sieden. Anschließend verdünnt man mit 1 400 ml Wasser, kühlt auf 0° C ab und saugt das kristalline Produkt ab. Zur Reinigung löst man das Kristallisat in Methylenchlorid und filtriert über eine Kieselgelsäure (Elutionsmittel: Methylenchlorid / Cyklohexan (9:11)).Die aus den vereinigten Eluaten erhaltene Substanz wird aus Isopropanol umkristallisiert. Ausbeute: 79 % der Theorie; Fp. 147 - 148° C.

3-Methyl-5,7-dinitrobenzothiopen

(Stufe D)

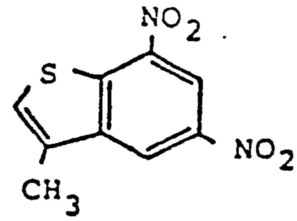

Eine Lösung von 0,8 Mol (2-Acetyl-4,6-dinitrophenyl-thio)essigsäure in 1 Liter Propionsäure wird bis zur Beendigung der $CO_2$-Entwicklung (ca. 4 Stunden) zum Sieden erhitzt.
Anschließend destilliert man die Propionsäure ab, nimmt den kristallinen Rückstand in Methylenchlorid auf und filtriert die Lösung über eine $Al_2O_3$-Schicht. Das nach dem Einengen des Filtrats erhaltene Produkt wird in Isopropanol ausgekocht, abfiltriert und getrocknet.
Ausbeute: 68 % der Theorie; Fp. 174 - 175° C.

(2-Acetyl-4,6-dinitrophenylthio)essigsäure
(Stufe C)

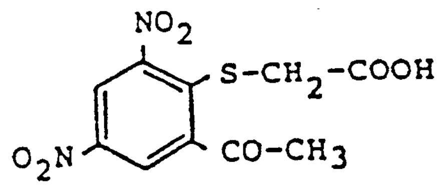

0,85 Mol 2-Chlor-3,5-dinitroacetophenon und 1,7 Mol Natriumhydrogencarbonat werden in einem Gemisch aus 650 ml Isopropanol und 250 ml Wasser suspendiert. Innerhalb von 10 Minuten tropft man 0,95 Mol Mercaptoessigsäure hinzu und rührt 2 Stunden bei 25° C, zuletzt 30 Minuten bei 45° C. Anschließend setzt man 1,8 Liter Eiswasser und 140 ml konzentrierte Salzsäure hinzu und saugt das Kristallisat ab. Das getrocknete Produkt wird aus Isopropanol umkristallisiert.
Ausbeute: 95 % der Theorie; Fp. 136 - 137° C.

2-Chlor-3,5-dinitroacetophenon
(Stufe B)

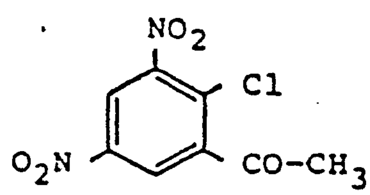

Eine Lösung von 1,1 Mol (2-Chlor-3,5-dinitrobenzoyl)-malonsäurediethylester in 500 ml Propionsäure wird nach Zusatz von 6 ml konzentrierter Schwefelsäure 3 Stunden zum Sieden erhitzt. Anschließend destilliert man die Propionsäure ab und versetzt den Rückstand mit 1,5 Liter Eiswasser. Das kristalline Reaktionsprodukt wird abgesaugt, mit Wasser gewaschen und getrocknet. Man löst das Produkt in Methylenchlorid und filtriert über eine 6 cm dicke $Al_2O_3$-Schicht. Das Filtrat wird nach Zusatz von 1 Liter Isopropanol eingeengt, das sich ausscheidende Kristallisat abgesaugt und getrocknet.
Ausbeute: 74 % der Theorie; Fp. 109 - 110° C.

(2-Chlor-3,5-dinitrobenzoyl)malonsäurediethylester
(Stufe A)

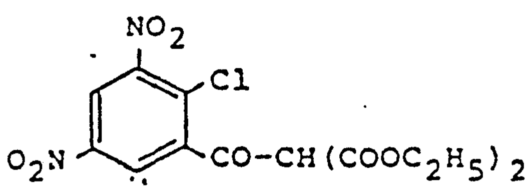

Zu einer Lösung von 1,7 Mol Ethoxymagnesiummalonsäurediethylester in 550 ml absolutem Toluol tropft man bei Raumtemperatur die Lösung von 1,14 Mol 2-Chlor-4,5-dinitrobenzoesäurechlorid (hergestellt aus 2-Chlor-3, 5-dinitrobenzoesäure durch 20-stündiges Kochen in $SOCl_2$) in 1,8 Liter absolutem Toluol, rührt 30 Minuten nach und gibt anschließend zu dem Ansatz 1 kg Eis sowie 150 ml 2n Schwefelsäure. Die organische Phase wird abgetrennt, mit Wasser neutral gewaschen und eingeengt. Nach Zugabe von 2 Liter Petrolether (Siedebereich 40-60°C) erfolgt Kristallisation.

Ausbeute: 95 % der Theorie; Fp. 71 - 72°C.

## Patentansprüche

1. 7,8,9,10-Tetrahydrothieno[3,2-e]pyrido [4,3-b] indole der allgemeinen Formel I

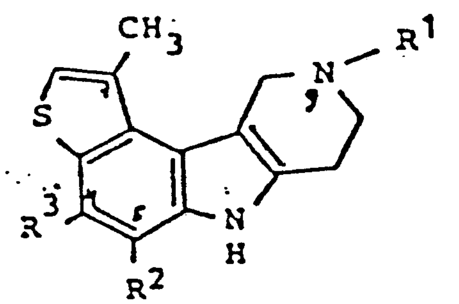

(I)

in welcher
$R^1$ für Wasserstoff oder geradkettigen oder verzweigten Alkyl mit 1 bis 4 C-Atomen steht, und
$R^2$ und $R^3$ für ein Wasserstoff und ein Halogen oder für zwei gleiche oder verschiedene Halogene stehen, sowie deren Säureadditionssalze.

2. Verbindungen nach Anspruch 1 der allgemeinen Formel I, in welcher
$R^1$ für Wasserstoff oder geradkettiges oder verzweigtes Alkyl mit 1 bis 4 C-Atomen steht, und
$R^2$ und $R^3$ in der Bedeutung Halogen für Fluor oder Chlor stehen.

3. Verbindungen der allgemeinen Formel I nach Anspruch 1, in welcher
$R^1$ für Methyl oder Ethyl,
$R^2$ für Fluor und
$R^3$ für Wasserstoff steht.

4. Arzneimittel enthaltend mindestens eine Verbindung der allgemeinen Formel I gemäß Anspruch 1.

5. Verwendung von Verbindungen der allgemeinen Formel I, gemäß Anspruch 1 zur Herstellung von Arzneimitteln zur beeinflussung des zentralen Nervensystems.

6. Verwendung von Verbindungen der Formel I gemäß Anspruch 1 zvr Herstellung von Antidepressive.

7 Verfahren zur Herstellung von Verbindungen der allgemeinen Formel I nach Anspruch 1, dadurch gekennzeichnet, daß man Hydrazinverbindungen der allgemeinen Formel II

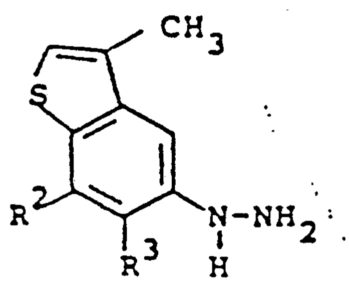

(II).

in welcher
$R^2$ und $R^3$ für ein Wasserstoff und ein Halogen oder für zwei gleiche oder verschiedene Halogene stehen, mit Piperidonen der allgemeinen Formel III

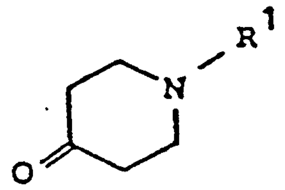

(III)

in welcher

$R^1$ für Wasserstoff oder geradkettiger oder verzweigter Alkyl mit 1 bis 4 C-Atomen steht bzw. mit Salzen dieser Piperidone mit Säuren in Gegenwart von inerten organischen Lösungsmitteln bei Temperaturen zwischen 20° C und 250° C umsetzt.

Verfahren nach Anspruch 7, dadurch gekennzeichnet, daß man

a) bei säureunempfindlichen Reaktionspartnern (II und III) deren Salze, vorzugsweise die Hydrochloride, in einem geeigneten Verdünnungsmittel einsetzt oder

b) bei säureempfindlichen Reaktionspartnern (II und III) oder die sich bevorzugt in Form ihrer Basen zu Verbindungen der allgemeinen Formel (I) umsetzen lassen bei höheren Temperaturen ohne Lösungsmittel oder in Gegenwart hochsiedender Lösungsmittel arbeitet.

9. 3-Methyl-5-hydrazino-benzothiophene der allgemeinen Formel II

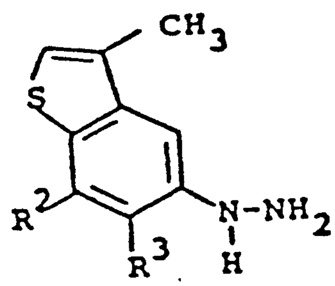

(II).

in welcher $R^2$ und $R^3$ für ein Wasserstoff und ein Halogen oder für zwei gleiche oder verschiedene Halogene stehen.

**Claims**

1. 7,8,9,10-Tetrahydrothieno[3,2-e]pyrido[4,3-b] indoles of the general formula

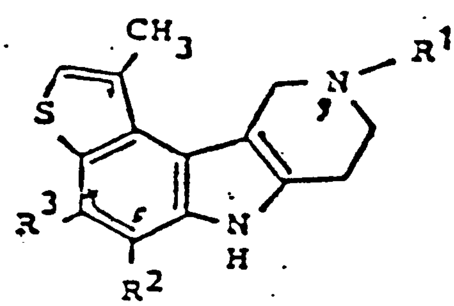

(I)

in which

$R^1$ represents hydrogen or straight-chain or branched alkyl with 1 to 4 C atoms and,

$R^2$ and $R^3$ represent a hydrogen and a halogen or two identical or difftrent halogens, and acid addition salts thereof.

2. Compounds according to Claim 1 of the general formula I, in which

$R^1$ represents hydrogen or straight-chain or

branched alkyl with 1 to 4 C atoms and

$R^2$ and $R^3$, when meaning halogen, represent fluorine or chlorine.

3. Compounds of the general formula I according to Claim 1, in which
$R^1$ represents methyl or ethyl,
$R^2$ represents fluorine and
$R^3$ represents hydrogen.

4. Medicaments containing at least one compound of the general formula I according to Claim 1.

5. Use of compounds of the general formula I according to Claim 1 for preparing medicaments, for influencing the central nervous system.

6. Use of compounds of the formula I according to Claim 1 for the preparation of antidepressants.

7. Process for the preparation of compounds of the general formula I according to Claim that hydrazine compounds of the general formula II

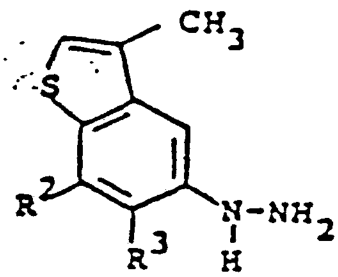

(II)

in which
$R^2$ and $R^3$ represent a hydrogen and a halogen or two identical or different halogens, are reacted with piperidones of the general formula III

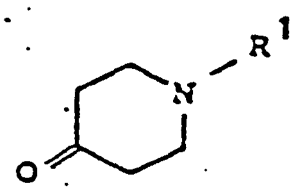

(III)

in which
$R^1$ represents hydrogen or straight-chain or branched alkyl with 1 to 4 C atoms,
or with salts of these piperidones with acids, in the presence of inert organic solvents at temperatures between 20° C and 250° C.

8. Process according to Claim 7, characterised in that a) in the case of acid-insensitive reactants (II and III), salts thertof, preferably the hydrochlorides, are used, in a suitable diluent, or b) in the case of acid-sensitive reactants (II and III) or which can preferably be reacted in tht form of their bases to give compounds of the general formula (I), the reaction is carried out at elevated temperatures without a solvent or in the presence of high-boiling solvents.

9. 3-Methyl-5-hydrazinobenzothiophenes of the general formula II

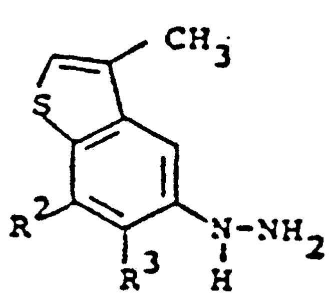

(II)

in which
$R^2$ and $R^3$ represent a hydrogen and a halogen or two identical or different halogens.

**Revendications**

1. 7,8,9,10-tétrahydrothiéno[3,2-e]pyrido [4,3-b]indols de formule générale I:

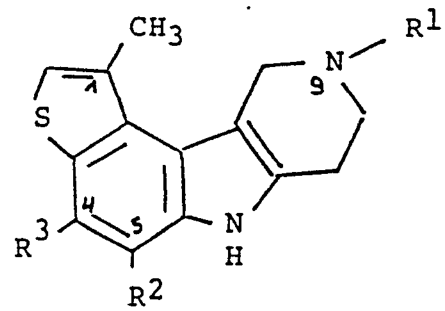

(I)

dans laquelle

$R^1$ représente de l'hydrogène ou un alcoyle à chaîne droite ou ramifiée ayant 1 à 4 atomes de carbone, et

$R^2$ et $R^3$ un hydrogène ou un halogène, ou deux halogènes identiques ou différents, ainsi que leurs sels d'addition d'acides.

2. Composés selon la revendication 1 de formule générale I, dans laquelle

$R^1$ représente de l'hydrogène ou un alcoyle à chaîne droite ou ramifiée ayant 1 à 4 atomes de carbone et

$R^2$ et $R^3$ dans la signification d'halogène sont du fluor ou du chlore.

3. Composés de formule générale I selon la revendication 1 dans lesquels

$R^1$ représente un méthyle ou éthyle,

$R^2$ du fluor et

$R^3$ de l'hydrogène.

4. Médicaments contenant au moins un composé de formule générale I selon la revendication 1.

5. Utilisation des composés de formule générale I selon la revendication 1 pour la préparation de médicaments influençant le système nerveux central.

6. Utilisation de composés de formule I selon la revendication 1 pour la préparation d'antidépressifs.

7. Procédé de fabrication de composés de formule générale I selon la revendication 1, caractérisé en ce qu'on fait réagir des composés d'hydrazine de formule générale II:

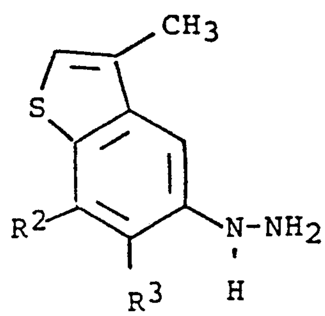

(II)

dans laquelle

$R^2$ et $R^3$ représentent un hydrogène ou un halogène, ou deux halogènes identiques ou différents, avec des pipéridones de formule générale III:

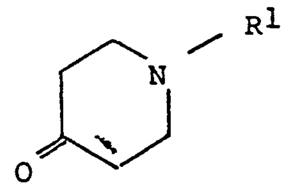

(III)

dans laquelle

$R^1$ représente de l'hydrogène ou un alcoyle à chaîne droite ou ramifiée ayant 1 à 4 atomes de carbone, ou avec des sels de ces pipéridones avec des acides en présence de solvants organiques inertes à des temperatures entre 20° C et 250° C.

8. Procédé selon la revendication 7, caractérisé en ce que

a) en cas de partenaires de réaction insensibles aux acides (II et III), on utilise leurs sels, de préférence les chlorhydrates, dans un diluant approprié, ou bien

b) dans le cas de partenaires de réactions (II et III) sensibles aux acides, ou qui réagissent de préférence sous forme de leurs bases pour donner des composés de formule générale (I), on opère à des températures plus élevées en l'absence de solvants ou en présence de solvants à point d'ébullition élevé.

9. 3-méthyl-5-hydrazino-benzothiophènes de formule générale II

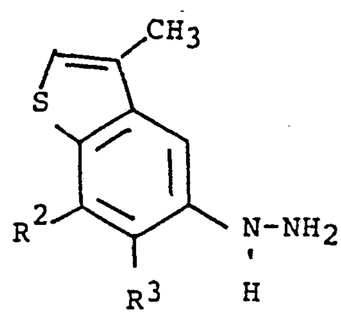

(II)

dans laquelle $R^2$ et $R^3$ représentent un hydrogène et un halogène, ou bien deux halogènes identiques ou différents.